# EUROPÄISCHE PATENTANMELDUNG

(11) **EP 2 859 981 A1**
(43) Veröffentlichungstag der Anmeldung: **15.04.2015**
(21) Anmeldenummer: 14183387.1
(22) Anmeldetag: 03.09.2014
(51) Int. Cl.: B23K 10/00, A61B 18/04, B23K 37/00, F16P 3/18, H05H 1/24, A61B 18/00

(54) **Handgerät und Verfahren zur Plasmabehandlung mit linken und rechten Handsensoren**

(30) Priorität: 10.09.2013 DE 102013109887
(71) Anmelder: Reinhausen Plasma GmbH, 93057 Regensburg (DE)
(72) Erfinder: Nettesheim, Stefan, 12205 Berlin (DE); Forster, Klaus, 93077 Bad Abbach (DE); Demirci, Arif, 93105 Tegernheim (DE)
(74) Vertreter: Reichert, Werner Franz

(57) **Zusammenfassung**

Die Erfindung betrifft ein Handgerät (1) und ein Verfahren zur Plasmabehandlung von Werkstücken (50). Das Handgerät (1) umfasst ein Gehäuse (60) zur Aufnahme einer Plasmaquelle (5). Der Plasmaquelle (5) wird von einer Gasversorgungseinheit (10) ein Gasstrom (13) zugeführt. Ferner ist eine Elektrodeneinheit (2) in der Plasmaquelle (5) angeordnet und über eine elektrische Leitung (21) mit einer Spannungsquelle (20) verbunden, so dass ein Plasmastrom (3) erzeugt werden kann. Der Plasmastrom (3) ist durch eine Austrittsdüse (4) des Gehäuses (60) auf ein Werkstück (50) richtbar. Ferner umfasst das Handgerät (1) ein Sensorsystem (40) zur Erhebung von Betriebsparametern (100). Das Sensorsystem (40) umfasst wenigstens einen Bedienersensoreinrichtung (41) zur Erhebung einer Position (101) eines Bedieners (70) relativ zur Plasmaquelle (5) und wenigstens einen Drucksensor (42) zur Erhebung eines Drucks (102) in der Gasversorgungseinheit (10). Eine Einrichtung (30) des Handgeräts (1) ist mit dem Sensorsystem (40) zur synchronisierten Erfassung der erhobenen Betriebsparameter (100) über Datenleitungen (31, 32, 36) kommunikativ verbunden. Desgleichen ist zumindest die Spannungsquelle (20) mit der Einrichtung (30) verbunden und steuer- bzw. regelbar.

## Beschreibung

Die Erfindung betrifft ein Handgerät zur Plasmabehandlung von Werkstücken. Das Handgerät umfasst ein Gehäuse zur Aufnahme einer Plasmaquelle. Der Plasmaquelle wird von einer Gasversorgungseinheit ein Gasstrom zugeführt. Ferner ist eine Elektrodeneinheit in der Plasmaquelle angeordnet und über eine elektrische Leitung mit einer Spannungsquelle verbunden, so dass ein Plasmastrom erzeugt werden kann. Der Plasmastrom ist durch eine Austrittsdüse des Gehäuses auf ein Werkstück richtbar.

Die Erfindung betrifft auch ein Verfahren zur Plasmabehandlung eines Werkstücks.

Die deutsche Patentanmeldung DE 10 2009 038 563 A1 offenbart ein Verfahren und eine Vorrichtung zur direkten Überwachung eines Plasmastrahls, insbesondere eines unter Atmosphärendruck erzeugten, kalten Plasmastrahls. Der Strom, der zwischen einer vor der Mündung angeordneten und dem Plasmastrahl ausgesetzten Stromabnehmers und dem geerdeten Gehäuse der Plasmaquelle fließt, wird dabei überwacht. Dieser Strom ist ein Maß für das Bestehen und die Beschaffenheit eines Plasmas. Sein Wert kann durch eine Ausgabeeinheit ausgegeben werden und einem Soll-Ist-Abgleich gegen nicht-flüchtig gespeicherte Grenzwerte unterzogen werden. Aus dem Soll-Ist-Abgleichen kann ein Signal generiert werden, das als Eingangssignal für die Regelung typischer Parameter des Plasmastrahls, wie z.B. der Stromstärke oder dem Gasfluss, dient. Die Vorrichtung ist an der Außenseite des Gehäuses einer bestehenden Plasmaquelle einfach nachrüstbar. Das Signal kann auch zur Erzeugung einer Warnmeldung dienen.

Die europäische Patentanmeldung EP 1 270 095 A2 offenbart ein Verfahren zur Überwachung einer Vorbehandlung einer zu verklebenden Oberfläche mit einem Plasmastrahl. Dabei wird die mit fortschreitender Plasmabehandlung ansteigende Temperatur der Oberfläche erfasst. Diese Oberflächentemperatur wird als Maß für die Qualität der Vorbehandlung herangezogen. Die Qualität der Vorbehandlung gilt als fehlerfrei, wenn die Oberflächentemperatur des Werkstücks bei der Plasmabehandlung in einem Bereich zwischen Mindestwert und einem Maximalwert geblieben ist. Die Oberflächentemperatur kann mit einem Infrarotsensor erfasst werden. Die Temperatur kann auch Eingangsgröße für einen Regelkreis sein. Geregelte Größen sind die Intensität des Plasmastrahls oder seine Positionierung relativ zur Oberfläche. Diese Intensität kann sowohl durch die Höhe der angelegten Spannung als auch durch die Stärke der Durchströmung mit Gas variiert werden.

Die internationale Patentanmeldung WO 2011/055368 A2 offenbart verschiedene Verfahren und medizinische Handgeräte zur Verschweißung von Gewebe eines Patienten mittels eines Plasmastrahls. Ein Gehäuse eines solchen Handgeräts enthält eine Batterie als Energiequelle einer RF-Zündvorrichtung, ein Gassubsystem aus einem Hochdruckgastank, einem Druckminderer und einen Gasflusskontroller und einer rohrförmigen Spitze, in der das Plasma erzeugt wird. Die Spitze kann über einen flexiblen Schlauch mit dem restlichen Gehäuse verbunden sein. Durch eine Öffnung der Spitze kann das Plasma als fokussierter Strahl austreten. Die Zündelektroden sind vorzugsweise bipolar ausgestaltet. Das Gewebe des Patienten kann gegen die RF-Zündvorrichtung geerdet sein. Am Gehäuse kann ein Bedienelement ausgebildet sein. Das Bedienelement dient der zur Steuerung der RF-Zündvorrichtung und des Gas-Subsystems oder dem Empfang eines Plasma-Feedbacksignals. Die Plasmatemperatur liegt unter 70°C bzw. kann durch einen Regelkreis darunter gehalten werden. Eingangsgrößen einer Steuereinheit zur Ausführung dieses Regelkreises sind RF-Leistung, RF-Impedanz, das Plasmaspektrum oder die Temperatur des Gewebes. Geregelt werden RF-Leistung und Gasfluss. Dem Plasmastrahl können aus einem Reservoir biokompatible Flüssigkeiten zugeführt werden.

Die U.S. Patentanmeldung US 2012/244290 A1 offenbart eine Vorrichtung zur insbesondere plasmagestützten Beschichtung von Werkstücken, die in einer Kammer gehaltert sind. Eine Spannungsquelle erlaubt das Anlegen einer Bias-Spannung an die Werkstücke. Ein Temperaturüberwachungssystem, bestehend aus seiner Vielzahl von Temperatursensoren, dient der Messung lokaler Temperaturen auf den Werkstücken. Die Temperatursensoren sind über optische Fasern mit einem Temperaturdatenprozessor kommunikativ verbunden und elektrisch voneinander isoliert, um elektrische Interferenzen der Temperaturmessung zu vermeiden.

Die U.S. Patentanmeldung US 2012/228271 A1 offenbart einen Schlauchaufbau mit Handgriff für ein mobiles Schweißgerät oder eine mobile Plasmafackel. Ein zur Plasmaerzeugung dienendes Gas wird einem Gastank entnommen. Der Handgriff wird mit Kühlflüssigkeit gekühlt, um den thermischen Stress gering zu halten, und um unterbrechungslos arbeiten zu können. Im Handgriff können insbesondere Fluss- und Temperatursensoren und Bedienelemente vorgesehen sein. Im Sinne der Betriebssicherheit ist das zu bearbeitende Werkstück mit einem Erdungskabel mit der Spannungsquelle verbunden. Die Spannungsquelle ist mit einem Kontroller zur Steuerung oder zur Regelung der für einen Schweißprozess relevanten, nicht näher benannten Parameter verbunden. Zweck der Regelung ist eine Stabilisierung der Arbeitsbedingungen des Schweißprozesses, nicht eine erhöhte Arbeits- und Betriebssicherheit und der Schutz vor thermischen und elektrischen Beschädigungen. Zur Kühlung ist ein sperriges und aufwendiges Kühlflüssigkeitssystem integriert, welches das Gerät insgesamt für den mobilen Einsatz sperrig macht.

Die Aufgabe der vorliegenden Erfindung besteht darin, ein flexibel einsetzbares, einfach aufgebautes und langlebiges Handgerät zu schaffen, mit dem auch ein ungelernter Bediener auf einfache, prozess- und betriebssichere Weise die Plasmabehandlung eines Werkstücks durchführen kann.

Die obige Aufgabe wird von einem Handgerät zur Plasmabehandlung von Werkstücken mit den Merkmalen des Anspruchs 1 gelöst.

Eine weitere Aufgabe der Erfindung ist, einflexibel einsetzbares Verfahren zu schaffen, mit dem auch ein ungelernter Bediener ein Werkstück auf einfache, prozess- und betriebssichere Weise plasmabehandeln kann.

Die obige Aufgabe wird von einem Verfahrenzur Plasmabehandlung von Werkstücken gemäß Anspruch 8 gelöst.

Die Erfindung betrifft ein Handgerät zur Plasmabehandlung von Werkstücken. Auf den Werkstücken können z.B. selektiv ausgewählte zu desinfizierende, zu aktivierende, zu beschichtende oder abzutragende Flächen, wie z. B. Löt- oder Klebestellen, plasmabehandelt werden. Statt einem Werkstück kann jedoch auch ein Patient, z.B. in einem Wundbereich, mit einem medizinisch geeignet kalten Plasma (z.B. unter 60°C, 65°C oder 70°C) behandelt werden. Insbesondere kann das Gerät so kompakt und leicht ausgestaltet sein, dass es von einem typischen Bediener mit einer oder zwei Händen zu führen ist. Das Handgerät ist insbesondere zur Führung durch einen menschlichen Bediener bestimmt. Alternativ kann der Bediener ein Roboter mit einem beweglichen Roboterarm sein.

Das Handgerät umfasst ein Gehäuse zur Aufnahme einer Plasmaquelle, eine Gasversorgungseinheit zur Zuführung eines Gasstroms zur Plasmaquelle und eine Spannungsquelle, die über eine elektrische Leitung mit einer Elektrodeneinheit der Plasmaquelle zur Erzeugung eines Plasmastroms verbunden ist. Im Gehäuse ist eine Austrittsdüse ausgebildet, aus der der Plasmastrom auf ein oder mehrere Werkstücke richtbar ist. Der Plasmastrom besteht vorzugsweise aus atmosphärischem und/oder kaltem Plasma.

Ferner umfasst das Handgerät ein Sensorsystem zur Erhebung von Betriebsparametern. Das Sensorsystem umfasst seinerseits wenigstens eine Bedienersensoreinrichtung zur Erhebung einer Position des Bedieners relativ zur Plasmaquelle und wenigstens einen Drucksensor zur Erhebung eines statischen und/oder dynamischen Drucks oder Partialdrucks in der Gasversorgungseinheit und/oder in der Plasmaquelle. Zudem umfasst das Handgerät eine Einrichtung, wie z.B. einen Controller, die mit dem Sensorsystem zur synchronisierten Erfassung der erhobenen Betriebsparameter und zumindest mit der Spannungsquelle zu ihrer Regelung über Datenleitungen kommunikativ verbunden ist. Einrichtung, Sensorsystem, Spannungsquelle und ggf. Gasversorgungseinheit bilden einen Regelkreis zur situationsbedingten Plasmabehandlung mit definierten Plasmaparametern. Ein Fachmann versteht unter einem Sensorsystem das Zusammenwirken von mehr als einem Sensor, um unterschiedliche Betriebsparameter zu erfassen.

Erfindungsgemäß kann das Sensorsystem des Handgeräts zusätzlich einen oder Kombinationen mehrerer zusätzlicher Sensoren umfassen. Zu diesen zusätzlichen Sensoren zählt beispielsweise mindestens einen Durchflusssensor zur Erhebung einer Flussrate des Gasstroms durch die Plasmaquelle, der in der Plasmaquelle oder in der Gasversorgungseinheit angeordnet ist. Ferner kann im Sensorsystem mindestens ein Temperatursensor zur Erhebung einer Temperatur der Plasmaquelle, der in oder an der Plasmaquelle angeordnet ist, umfasst sein. Denkbar ist auch ein Temperatursensor zur Messung einer Oberflächentemperatur, zumindest des plasmabehandelten Bereichs, auf dem Werkstück. Das Sensorsystem kann auch mindestens eine Kamera zur Erfassung zumindest eines Bereichs des Werkstücks umfassen. Ebenso kann mindestens ein kinematischer Sensor zur Erhebung zumindest einer kinematischen Größe des Handgeräts relativ zum Werkstück umfasst sein. Kinematische Größen sind Positionen, translatorische oder rotatorische Geschwindigkeiten und Beschleunigungen bezüglich einer, zwei oder drei Raumdimensionen bzw. Raumachsen. Die Winkelerfassung ist deshalb von Bedeutung, weil die Homogenität und Flächendosis der Plasmabehandlung vom Winkel abhängen, unter dem der Plasmastrahl auf das Werkstück gerichtet wird. Im erfindungsgemäßen Handgerät kann ein Beschleunigungssensor einen Geschwindigkeitssensor oder einen Positionssensor substituieren, indem die Einrichtung die Betriebsparameter, wie z.B. die Beschleunigung, zeitaufgelöst erfasst und zu den Betriebsparametern, wie z.B. Geschwindigkeit oder Position, bezüglich einer Ausgangsposition numerisch auf integriert. Die Einrichtung kann vorzugsweise als Computer oder Mikrocontroller ausgestaltet sein.

Die Einrichtung oder das Sensorsystem kann insbesondere zur Durchführung je eines Soll-Ist-Abgleichs für jeden vom Sensorsystem erhobenen Betriebsparameter ausgebildet sein.

Das Handgerät kann zudem eine Zielvorrichtung umfassen, die zur Markierung eines Ortes und/oder eines Bereichs auf dem Werkstück dient, auf den der Plasmastrom gerichtet ist. Im einfachsten Fall kann die Zielvorrichtung ein Aufsatz über dem Plasmaauslass sein, der auf das Werkstück aufgesetzt werden kann. Die Länge des Aufsatzes bestimmt somit den Arbeitsabstand von Austrittsdüse zu Werkstück.

In einer weiteren Ausführungsform kann die Zielvorrichtung wenigstens einen Projektor umfassen, mit dem wenigstens ein Lichtsignal auf das Werkstück projizierbar ist, vorzugsweise an den Ort oder auf den Bereich des Werkstückes, auf den der Plasmastrahl wirkt.

Bei einer besonderen Ausführungsform des wenigstens einen Projektors können die Lichtsignale zeitlich, farblich und/oder bezüglich der Form moduliert werden. Beispielsweise können die Lichtsignale grün sein, solange ein oder mehrere Betriebsparameter innerhalb eines jeweiligen Soll-Bereichs liegen. Nähert oder überschreitet ein Betriebsparameter den Sollbereich innerhalb eines jeweiligen Toleranzbereiches, kann das Lichtsignal beispielsweise auf orange umschalten. Überschreitet ein Betriebsparameter seinen Toleranzbereich, kann das Lichtsignal beispielsweise auf rot umschalten. In diesem Fall wäre der Projektor als Leucht-Dioden-Array oder Laser-Dioden-Array ausgestaltet. Statt einer farblichen Kenntlichmachung kann das Überschreiten auch durch ein charakteristisch blinkendes Lichtsignal gekennzeichnet werden. Dies hat gegenüber einer farblichen Kenntlichmachung den Vorteil, dass z.B. nur ein Leuchtdidodentyp pro Projektor benötigt wird. Ferner kann die Wellenlänge des Lichtsignals so gewählt werden, dass es auf dem Werkstück gut sichtbar ist.

Ferner kann am Handgerät mindestens eine Bedieneranzeige vorgesehen sein. Mittels einer solchen Bedieneranzeige können optische, akustische und/oder vibratorische Bedienerhinweise bezüglich des Betriebszustandes des Handgeräts ausgegeben werden. Der Betriebszustand wird im Sinne der Erfindung vor allem durch das Über- oder Unterschreiten von oberen bzw. unteren Schwellwerten durch Betriebsparameter bestimmt. Zu diesen Betriebsparametern zählen beispielsweise der Arbeitsabstand oder eine Geschwindigkeit des Plasmaauslasses bezüglich der Oberfläche des Werkstücks, die Winkelausrichtung des Handgeräts relativ zur lokalen Oberflächennormale des Werkstücks, die Temperatur am Plasmaauslass oder auf dem Werkstück und der statische oder dynamische Druck in der Plasmaquelle.

Das Über- und/oder Unterschreiten von Schwellwerten kann beispielsweise mit verschiedenen, insbesondere jeweils charakteristischen Warntönen unterlegt werden. Der Bedienerhinweis kann auch als Vibration erfolgen. Die Vibration kann beispielsweise von einem Vibrator erzeugt werden, der mit dem Gehäuse oder einem Bedienelement des Handgeräts mechanisch gekoppelt ist.

Die Bedienersensoreinrichtung kann zudem als Bedienelement zur Eingabe von Bediensignalen durch den Bediener ausgebildet sein. Beispielsweise kann eine solche Bedienersensoreinrichtung mechanische Druck- oder Drehregler oder kapazitive Bedienelemente umfassen. Beispielsweise kann ein erstes Bedienelement den Gasfluss regeln und ein zweites Bedienelement die Spannung der Spannungsquelle einstellen. Mittels eines Bedienelements, wie z.B. einem kapazitiven Touchpad, können manuell ein oder mehrere voreingestellte Plasmaparameter oder Plasmaleistungen ausgewählt werden. Zu diesen Plasmaparametern zählen die Elektronen- und Ionentemperaturen, der Ionisationsgrad und Druck und Strömungsverhältnisse im Plasmastrom.

Die Bedienersensoreinrichtung kann insbesondere als ein erster Handsensor zur Erhebung der Position einer rechten Hand und als ein zweiter Handsensor zur Erhebung der Position einer linken Hand des Bedieners am Gehäuse ausgestaltet sein. Vorzugsweise sind der erste und zweite Handsensor so am Gehäuse angeordnet und voneinander beabstandet, dass sie nicht mit einer gewöhnlichen menschlichen Hand (Länge ca. 15-30 cm) bedient werden können. Gemäß einer besonderen Ausführungsform des erfindungsgemäßen Handgeräts gibt die Einrichtung die Spannung und/oder den Gasfluss nur solange frei, wie sie Signale vom ersten und zweiten Handsensor erfasst. Durch die Festlegung der Position beider Hände des Bedieners ist sichergestellt, dass keine seiner Hände in den Plasmastrom gerät und verletzt wird.

Die Einrichtung kann insbesondere mit einer Datenbank zur Speicherung von Betriebsparametern und/oder aus Betriebsparametern abgeleiteten und/oder auf den Bediener bezogenen Metadaten kommunikativ verbunden sein. Aus den Betriebsparametern ergibt sich beispielsweise ein Bearbeitungsprofil bzw. es ist daraus ableitbar. Bedienerbezogene Metadaten sind beispielsweise eine Kennung des Bedieners oder des von ihm bearbeiteten Werkstücks, der Zeitraum der Bearbeitung und ggf. Umgebungseinflüsse, wie z.B. Außentemperatur oder Luftfeuchtigkeit. Die kommunikative Verbindung kann kabelgebunden oder drahtlos sein oder in einem portablen Speichermedium wie z.B. einer SD-Karte oder einem USB-Stick bestehen. Durch die Verknüpfung von Bearbeitungsprofilen aus Bedienerparametern und bedienerbezogenen Metadaten sowie die Überwachung und Regelung der Plasmabehandlung kann mit dem Handgerät ein Produktleitverfahren (bzw. Manufacturing Execution System (MES)) implementiert werden. Beispielsweise wird als elektronische Signatur protokolliert, wann wer ein bestimmtes Werkstück entlang welcher Trajektorie mit welchen Plasmaparametern behandelt hat. Viele arbeitsteilige Fertigungsprozesse mit zahlreichen konsekutiven Prozessschritten erfordern derartige elektronische Signaturen für die Prozesssicherheit und das Qualitätsmanagement.

Die Einrichtung kann über eine Datenleitung mit der Gasversorgungseinheit zur Regelung des Gasstroms kommunikativ verbunden sein. So können die Plasmaparameter eingestellt werden, indem die Einrichtung den Gasstrom und die Spannung abgestimmt regelt. Die Gasversorgungseinheit ist beispielsweise als ein regelbares Gebläse und/oder eine Gasdruckflasche mit einem regelbaren Ventil implementiert. Vorzugsweise ist die Gasversorgungseinheit im Gehäuse des Handgeräts integriert oder am Gehäuse angebracht. Wird die Spannungsquelle aus einer ebenfalls im oder am Gehäuse angeordneten Batterie gespeist, ist das Handgerät nicht mit einem Kabel oder Schlauch versehen, ist es portabel und somit flexibel einsetzbar. Alternativ kann auch ein Anschlussventil für eine Druckgasleitung am Gehäuse vorgesehen sein. Aus der Druckgasleitung oder aus der Gasdruckflasche kann das Handgerät mit verschiedenen Gasen, wie Druckluft, Inertgasen (wie Argon oder Stickstoff) oder chemisch reaktiven Gasen, gespeist werden. Ferner kann dem Gasstrom durch eine Mischvorrichtung ein flüssiger oder pulverförmiger Zusatzstoff aus einem Reservoir beigesetzt oder eingedüst und dann ggf. plasmaaktiviert werden. Der Zusatzstoff kann beispielsweise ein Flussmittel für einen nachfolgenden Lötprozess, ein Reduktions- oder Oxidationsmittel, eine medizinisch wirksame Substanz oder ein Beschichtungsmaterial sein.

Die Erfindung betrifft ferner ein Verfahren zur Plasmabehandlung eines Werkstücks. Zur eigentlichen Plasmabehandlung wird mit einer Plasmaquelle, z.B. eines Handgeräts, ein Plasmastrom erzeugt. Der so erzeugte Plasmastrom wird von einem Bediener des Handgeräts auf ein Werkstück gerichtet. Zumindest während der Plasmabehandlung werden mittels eines Sensorsystems mindestens zwei Betriebsparameter erhoben. Dabei wird zumindest mittels wenigstens einer Bedienersensoreinrichtung des Sensorsystems eine Position des Bedieners relativ zur Plasmaquelle und einen mittels wenigstens eines Drucksensors des Sensorsystems ein Druck in einer Gasversorgungseinheit der Plasmaquelle erfasst. Die so erhobenen Betriebsparameter werden synchronisiert an eine Einrichtung übermittelt. Die Synchronisierung erlaubt die zeitliche Korrelation der Betriebsparameter. Durch die Einrichtung werden anhand der erhobenen Betriebsparameter Steuersignale zum Freigeben und/oder Regeln einer Spannungsquelle der Plasmaquelle und ggf. der Gasversorgungseinheit erzeugt.

Mittels des Sensorsystems kann zumindest ein zusätzlicher Betriebsparameter erhoben werden. Zu diesen zusätzlichen Betriebsparametern zählt beispielsweise mindestens eine Flussrate des Gasstroms mittels mindestens eines in der Plasmaquelle oder in der Gasversorgungseinheit angeordneten Durchflusssensors. Auch kann mindestens eine Temperatur der Plasmaquelle mittels mindestens eines in oder an der Plasmaquelle angeordneten Temperatursensors erfasst werden. Denkbar ist auch die Erfassung der Temperatur des plasmabehandelten Bereichs des Werkstücks durch z.B. einen Infrarotsensor, um ein Überhitzen des Werkstücks zu detektieren oder zu vermeiden. Ferner kann ein optisches Abbild zumindest eines Bereiches des Werkstücks mittels einer Kamera des Handgeräts erzeugt werden. In diesem Fall ist die Einrichtung vorzugsweise zur Ausführung bilderkennender Verfahren ausgelegt, so dass sie bestimmte Strukturen auf dem Werkstück identifizieren und lokalisieren kann.

Es ist auch ein "teach-in" Verfahren denkbar, dass ein Musterbild einer Struktur zunächst im Hanggerät abspeichert wird. Diese Struktur wird dann auf den Werkstücken mittels der Kamera des Handgeräts gesucht und ausgewertet. Nur bei entsprechender Ähnlichkeit wird dann der Bearbeitungsprozess ausgelöst. Solche Strukturen können beispielsweise Marker, elektronische Bauteile auf einer Platine oder Wundnähte sein.

Die Einrichtung kann anhand der Position der Strukturen einen mit dem Handgerät zu bearbeitenden Bereich lokalisieren. Form und Soll-Position der Strukturen und des zu bearbeitenden Bereichs sind vorab in der Einrichtung einzuprogrammieren. Insbesondere kann mindestens ein kinematischer Betriebsparameter des Handgeräts relativ zum Werkstück mittels mindestens eines am Handgerät angeordneten kinematischen Sensors erfasst werden. Gegenstand der Regelung durch die Einrichtung kann beispielsweise sein, die Plasmaparameter konstant zu halten. Werden kinematische Sensoren in den Regelkreis einbezogen, kann die Einrichtung auch die auf das Werkstück wirkende Plasmaleistung pro Fläche konstant halten, um einen menschlichen Bediener bei einer homogenen Plasmabehandlung zu unterstützen.

Bei geeignet eingestellter Schärfentiefe der Kamera ist es auch möglich, den korrekten Bearbeitungsabstand aus den Bilddaten zu ermitteln. Wird zusätzlich ein Bilderkennungsverfahren auf die Bilddaten angewandt, kann so die vollständige Rauminformation für die zu erfolgende Plasmabearbeitung ermittelt oder auch fortlaufend überwacht werden. Der eingestellte Schärfentiefebereich kann dazu verwendet werden, dass sich das Handgerät während der Bearbeitung des Werkstücks in dem durch den Schärfentiefebereich definierten Sollarbeitsabstand befindet.

Erfindungsgemäß kann für die erhobenen Betriebsparameter je ein Soll-Ist-Abgleich durchgeführt werden. Die Einrichtung erzeugt Steuersignale, wenn beim jeweiligen Soll-Ist-Abgleich der entsprechende Betriebsparameter länger als ein jeweiliges Zeitintervall entweder einen jeweiligen minimalen oder maximalen Schwellwert überschreitet respektive unterschreitet oder innerhalb eines jeweiligen Soll-Intervalls liegt. Ein Soll-Intervall wird durch einen jeweiligen oberen und unteren Schwellwert definiert.

Insbesondere können dem Bediener, vermittels einer Bedieneranzeige des Handgeräts, Bedienerhinweise angezeigt werden. Die Bedienerhinweise beziehen sich beispielsweise auf die Betriebsbereitschaft des Handgeräts, auf zumindest einen erhobenen Betriebsparameter und/oder auf das Überschreiten oder Unterschreiten von oberen respektive unteren Schwellwerten durch mindestens einen der erhobenen Betriebsparameter.

Ferner kann mittels einer Zielvorrichtung des Handgeräts ein Ort und/oder ein Bereich auf dem Werkstück markiert werden, auf den der Plasmastrom gerichtet ist. Die Zielvorrichtung ist im einfachsten Fall ein mechanischer Aufsatz über einer Austrittsdüse für den Plasmastrahl. Vorzugsweise umfasst die Zielvorrichtung jedoch wenigstens einen Projektor. Jeder Projektor kann mindestens ein Lichtsignal auf das Werkstück projizieren.

Insbesondere kann durch zeitabhängige Soll-Intervalle kinematischer Betriebsparameter eine Soll-Trajektorie über dem Werkstück definiert werden. Ein Anfangspunkt bzw. örtlicher Referenzpunkt auf dem Werkstück kann vom Bediener, von der Kamera oder von einem Ortssensor erfasst werden. Der Bediener hat das Handgerät auf einer Ist-Trajektorie entlang der Soll-Trajektorie über das Werkstück zu führen. Abweichungen der Plasmabehandlung entlang dieser Trajektorie, z.B. bezüglich der Plasmaparameter, der Vorschubgeschwindigkeit des Handgeräts, der Flächendosis des auf das Werkstück wirkenden Plasmas etc., können dem Bediener von der Einrichtung rückgemeldet und/oder protokolliert werden. Die Schwellwerte z.B. bezüglich der Flächendosis des Plasmas oder der maximalen Oberflächentemperatur auf dem Werkstück können mittels entsprechender Programmierung der Einrichtung örtlich moduliert werden, um beispielsweise ein hitzeempfindliches Bauteil auf der Trajektorie zu schonen.

Gegebenenfalls ist die Soll-Trajektorie auf dem Werkstück für den menschlichen Bediener nicht oder nur schwer erkennbar. Daher sieht die Erfindung vor, dass die Zielvorrichtung die Soll-Trajektorie zumindest abschnittsweise auf das Werkstück projiziert. Der so markierte Abschnitt kennzeichnet z.B. den Bereich, die Vorschubrichtung und ggf. Vorschubgeschwindigkeit, mit der der Bediener das Handgerät zu führen hat. So kann auch ein ungelernter Bediener ein Werkstück einer komplexen Plasmabehandlung unterziehen.

Zur gesteigerten Betriebs- und Prozesssicherheit sieht das erfindungsgemäße Verfahren vorzugsweise vor, dass die Einrichtung Steuersignale an die Spannungsquelle nur ausgibt, solange kein erhobener Betriebsparameter länger als ein jeweiliges Zeitintervall außerhalb eines jeweiligen Soll-Intervalls liegt oder einen jeweiligen minimalen oder maximalen Schwellwert unterschreitet respektive überschreitet.

Die Einrichtung kann erfindungsgemäß auch Steuersignale für eine Gasversorgungseinheit der Plasmaquelle erzeugen. Ebenso kann sie die Spannungsquelle und die Gasversorgungseinheit in Abhängigkeit der jeweils in der Gasversorgungseinheit und der Spannungsquelle vorherrschenden Betriebsparameter regeln.

Mittels eines internen Taktgebers kann die Einrichtung die Spannungsquelle und die Gasversorgungseinheit derart zeitlich abgestimmt steuern, dass der Gasstrom die Plasmaquelle während einer Vorlaufzeitspanne vor einer Freigabe der Spannungsquelle und/oder während einer Nachlaufzeitspanne nach Abschalten der Spannungsquelle durchfließt.

Die Betriebsparameter und/oder aus Betriebsparametern abgeleiteten und/oder auf den Bediener bezogenen Metadaten an eine kommunikativ mit der Einrichtung verbundenen Datenbank gespeichert werden. Daraus kann für jedes plasmabehandeltes Werkstück bzw. seine Charge eine elektronische Signatur erzeugt werden.

Eine wesentliche Grundidee der Erfindung beruht auf der seriellen Verschaltung (UND-Verknüpfung) verschiedener Sensoren in einem Handgerät zur Plasmabehandlung. Die Sensoren erfassen die für die Arbeits- und Betriebssicherheit relevanten Betriebsparameter. Weichen die Betriebsparameter von vorgegebenen Schwellwerten ab, wird die Spannungsversorgung des Plasmastroms von der Einrichtung des erfindungsgemäßen Handgeräts entweder gesperrt oder freigegeben. In der Korrelation verschiedener Betriebsparameter ist zusätzliche Information über die Plasmabehandlung enthalten.

Diese Einrichtung verknüpft gemessene Betriebsparameter, wie z.B. Gasdruck und -fluss, die relative Position der Austrittsdüse zum Werkstück und/oder zum Bediener und die Position einer oder beider Hände des Bedieners am Handgerät. Gemäß einem festgesetzten zeitlichen Ablauf gibt die Einrichtung die Gasversorgungseinheit und/oder die Spannungsquelle frei oder sperrt sie.

Ein typischer Ablauf sieht vor, die Handposition(en) des Bedieners zu erfassen, dann den Hochlauf der Gasversorgungseinheit freizugeben, ab dann den Druck und Fluss des Gasstromes sowie die korrekte Position der Austrittsdüse zum Bearbeitungsort abzufragen. Nach einer Zeitverzögerung von typischerweise 1 s nach Hochlauf der Gasversorgungseinheit erfolgt die Freigabe der Spannungsquelle. Beim Sperren der Spannungsquelle erfolgt ein Nachlauf der Gasversorgungseinheit von typischerweise 5 s zur Kühlung und zum elektrostatischem Ladungsausgleich in der Plasmaquelle.

Dieser Sicherheitsmechanismus erhöht nicht nur die Arbeits- und Prozesssicherheit, sondern schont zudem die Plasmaquelle und trägt folglich zu einer längeren Lebensdauer des Handgeräts bei. Eine erhöhte Arbeitssicherheit erlaubt es zudem, die bisher nur geringe, aus Sicherheitsgründen begrenzte Leistung handgeführter Plasmaquellen zu erhöhen. Dadurch können Fertigungsabläufe effizienter gestaltet oder neue Anwendungsgebiete für handgeführte Plasmaquellen erschlossen werden. Ferner kann die erfindungsgemäße Einrichtung die Einstellung und Reglung der Plasmaparameter übernehmen. Durch diese Teilautomatisierung kann auch ein ungelernter Bediener eine grundsätzlich komplexe Plasmabehandlung auf einfache Weise ausführen. Vor allem in arbeitsteiligen Fertigungsprozessen gewinnt die vorzugsweise elektronische Dokumentation jedes einzelnen Prozessschrittes stetig an Bedeutung. Das erfindungsgemäße Handgerät kann dieser Erfordernis Rechnung tragen, indem relevante Betriebsparameter und/oder auf den Bediener bezogene Metadaten in ihrer Einrichtung oder einer mit ihr verbundenen Datenbank protokolliert werden. Auf diese Weise sind Fehlerquellen im Gesamtprozess nachvollziehbarer und einfacher auszuräumen. Manche Absatzmärkte bleiben ohne solche Prozessprotokolle bzw. elektronische Signaturen verschlossen. Insbesondere kann ein erfindungsgemäß teilautomatisiertes Handgerät die korrekte Ausführung der Plasmabehandlung überwachen und dem Bediener dazu ein direktes Feedback bzw. Hilfestellung geben.

Weitere Vorteile und vorteilhafte Ausgestaltungen der Erfindung sind Gegenstand der nachfolgenden Figuren und ihrer zugehörigen Beschreibungsteile.

Es zeigen im Einzelnen:
- **Figur 1**: eine schematische Ansicht einer Ausführungsform eines erfindungsgemäßen Handgeräts zur Plasmabehandlung von Werkstücken;
- **Figur 2**: eine schematische Seitenansicht einer weiteren Ausführungsform des erfindungsgemäßen Handgeräts;
- **Figur 3**: eine schematische Ansicht einer Ausführungsform eines Gehäuses für das erfindungsgemäße Handgerät;
- **Figur 4A**: eine schematische Ansicht einer weitere Ausführungsform des Gehäuses für das erfindungsgemäße Handgerät;
- **Figur 4B**: eine schematische Ansicht der Ausführungsform des Gehäuses für das erfindungsgemäße Handgerät aus Figur 4A aus einem anderen Blickwinkel;
- **Figur 5**: eine schematische Ansicht einer weiteren Ausführungsform des Gehäuses für das erfindungsgemäße Handgerät;
- **Figur 6A**: eine schematische Ansicht auf eine Zielvorrichtung des erfindungsgemäßen Handgeräts zur Markierung eines plasmazubehandelnden Bereichs auf einem Werkstück;
- **Figur 6B**: eine schematische Draufsicht auf das mit der Zielvorrichtung des Handgeräts gemäß Figur 6A mit Bedienerhinweisen markierten Werkstücks;
- **Figur 7**: eine schematische Darstellung einer erfindungsgemäß markierten und plasmazubehandelnden Soll-Trajektorie auf einem Werkstück; und
- **Figur 8**: die Darstellung des erfindungsgemäßen Verfahrens zur Plasmabehandlung von Werkstücken anhand schematischer Zeitdiagramme für verschiedene Betriebsparameter und Steuersignale.

Für gleiche oder gleich wirkende Elemente der Erfindung werden identische Bezugszeichen verwendet. Die dargestellten Ausführungsbeispiele stellen lediglich Möglichkeiten dar, wie ein erfindungsgemäßes Handgerät oder ein Verfahren zur Plasmabehandlung von Werkstücken ausgestaltet sein können bzw. die offenbarten Merkmale kombiniert werden können.

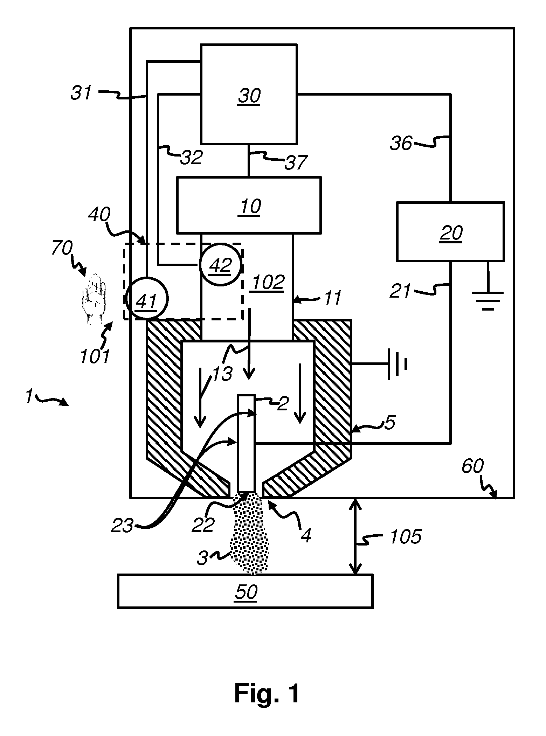

**Figur 1** zeigt eine schematische Ansicht einer Ausführungsform des erfindungsgemäßen Handgeräts 1 zur Plasmabehandlung von Werkstücken 50. Ein Gehäuse 60 des Handgeräts 1 nimmt eine Plasmaquelle 5, eine Gasversorgungseinheit 10 und eine Spannungsquelle 20 auf. Die Gasversorgungseinheit 10 steht über einen Gasversorgungskanal 11 mit der Plasmaquelle 5 in fluider Verbindung. Sie dient der Zuführung eines Gasstroms 13 zur Plasmaquelle 5, der eine Elektrode 2 der Plasmaquelle 5 überströmt. Gleichzeitig bewirkt der Gasstrom 13 die Kühlung insbesondere der Plasmaquelle 5. Die Spannungsquelle 20 ist über mindestens eine elektrische Leitung 21 mit der Elektrodeneinheit 2 verbunden. Durch Anlegen einer Spannung an der Elektrodeneinheit 2 wird aus dem Gasstrom 13 ein Plasmastrom 3 gezündet. Durch eine Austrittsdüse 4 des Gehäuses 60 ist der Plasmastrom 3 auf ein Werkstück 50 richtbar.

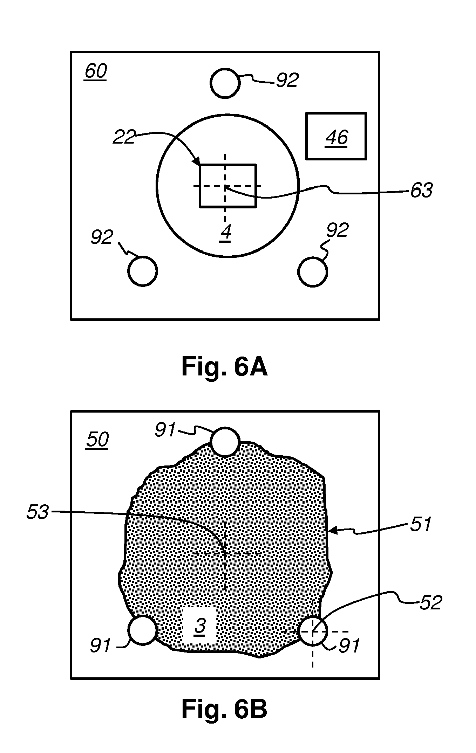

Vorzugsweise ist die Elektrodeneinheit 2 ein piezoelektrischer Transformator. In diesem Fall sind zwei Sekundärseiten 23 der Elektrodeneinheit 2 über je eine elektrische Leitung 21 mit der Spannungsquelle 2 verbunden (nicht dargestellt). Aufgrund des bezüglich der Spannungsamplitude hohen Transformationsverhältnisses kann durch sekundärseitiges Anlegen einer niedrigen Wechselspannung, wie sie z.B. aus einer typischen portablen Batterie 24 speisbar ist, vermittels mechanischer Schwingungen im Piezomaterial eine hohe Wechselspannung an einer Primärseite 22 des piezoelektrischen Transformators erzeugt werden. An der Primärseite 22 wird aus dem Gasstrom 13 vermittels dieser Hochspannung der Plasmastrom 3 gezündet. Vorzugsweise ist die Primärseite 22 der Elektrodeneinheit 2 kontaktfrei und zentriert in der Austrittsdüse 4 angeordnet (siehe Figur 6A).

Das Handgerät 1 umfasst ferner ein Sensorsystem 40 zur Erhebung von Betriebsparametern 100. Das Sensorsystem 40 umfasst wenigstens eine Bedienersensoreinrichtung 41 zur Erhebung einer Position 101 eines Bedieners 70 relativ zur Plasmaquelle 5. Die Bedienersensoreinrichtung 41 dient insbesondere der Prüfung, ob der Plasmastrahl 3 auf den Bediener 70 des Handgeräts gerichtet ist. Ferner umfasst das Sensorsystem 40 wenigstens einen Drucksensor 42 zur Erhebung eines statischen und/oder dynamischen Drucks 102 in der Gasversorgungseinheit 10, im Gasversorgungskanal 11 oder in der Plasmaquelle 5. Falls der Gasstrom 13 aus mehreren Gaskomponenten zusammengesetzt ist, können Drucksensoren 42 auch einen oder mehrere ausgewählte Partialdrücke erfassen. Eine Einrichtung 30 des Handgeräts 1 ist mit dem Sensorsystem 40 zur synchronisierten Erfassung der erhobenen Betriebsparameter 100 über Datenleitungen 31, 32, 36 kommunikativ verbunden. Ferner ist die Einrichtung 30 über Datenleitungen 36 und 37 kommunikativ mit der Spannungsquelle 20 respektiveder Gasversorgungseinheit 10 verbunden. Die Einrichtung steuert die Spannungsquelle 20 und die Gasversorgungseinheit 10 bzw. einen Regelkreis aus Spannungsquelle 20, Gasversorgungseinheit 10 und Sensorsystem 40.

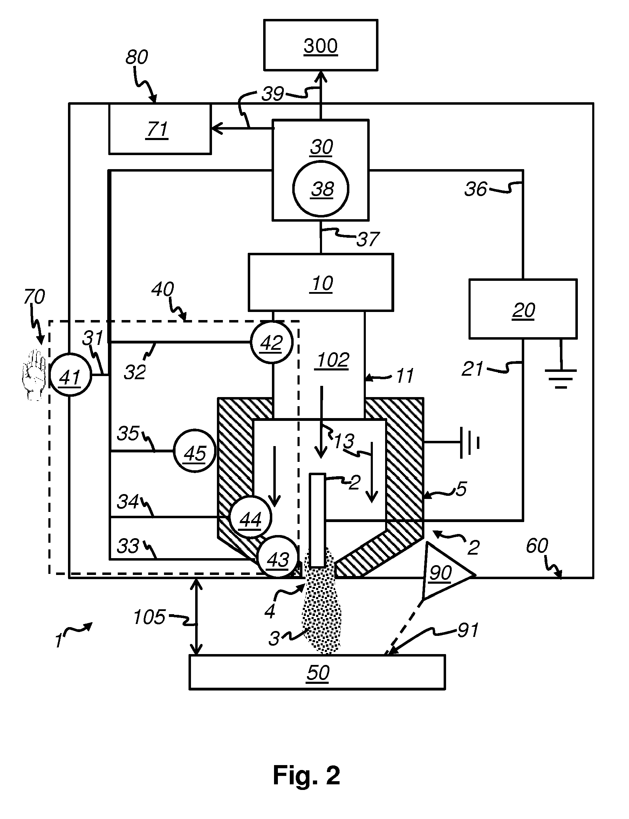

**Figur 2** zeigt eine schematische Ansicht einer weiteren Ausführungsform des erfindungsgemäßen Handgeräts 1. Sie umfasst alle Merkmale der in Figur 1 gezeigten Ausführungsform. Zusätzlich zu einer Bedienersensoreinrichtung 41 und einem Drucksensor 42 umfasst das Sensorsystem 40 einen z.B. an der Austrittsdüse 4 angeordneten Durchflusssensor 43, einen z.B. in der Plasmaquelle 5 angeordneten Temperatursensor 44 und mehrere kinematische Sensoren 45. Alle Sensoren sind über jeweilige Datenleitungen 31, 32, 33, 34, 35 mit der Einrichtung 30 verbunden. Die Einrichtung 30 umfasst einen internen Taktgeber 38, so dass sie die Spannungsquelle 20 und die Gasversorgungseinheit 10 zeitlich abgestimmt steuern kann. Auf diese Weise kann der Gasstrom 13 die Plasmaquelle 5 während einer Vorlaufzeitspanne vor einer Freigabe der Spannungsquelle 20 durchfließen. Damit ist sichergestellt, dass sich der Gasstrom 13 stabilisiert, bevor ein Plasmastrom 3 aus ihm gezündet wird. Sicherheitshalber und zur Kühlung nach einer Plasmabehandlung kann der Gasstrom 13 die Plasmaquelle 5 auch während einer Nachlaufzeitspanne nach Abschalten der Spannungsquelle 20 durchfließen. Über weitere Datenleitungen 39 ist die Einrichtung 30 zudem mit einer internen oder externen Datenbank 300 und/oder einer Bedieneranzeige 71 zur Anzeige von Bedienerhinweisen 80 verbunden. Ferner trägt das Gehäuse 60 eine Zielvorrichtung 90, die ebenfalls Bedienerhinweise 80 wie z.B. Lichtsignale 91 auf das Werkstück 50 projiziert.

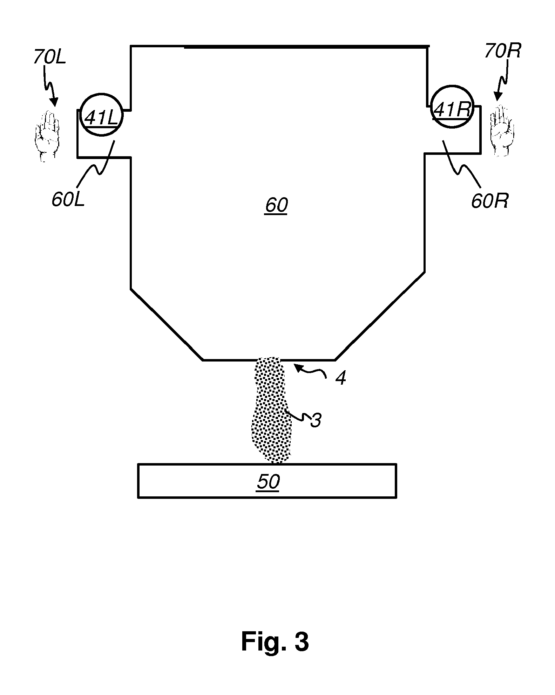

**Figur 3** zeigt eine schematische Ansicht einer Ausführungsform eines Gehäuses 60 des erfindungsgemäßen Handgeräts 1. Am Gehäuse 60 sind ein linker Handgriff 60L und ein rechter Handgriff 60R ausgebildet. Dem Fachmann ist klar, dass diese hier nur schematisch dargestellte Ausführungsform bezüglich der Form und Anordnung der Handgriffe 60L, 60R an die ergonomischen Erfordernisse eines Handgeräts 1 anzupassen sind. Der linke Handgriff 60L und der rechte Handgriff 60R tragen einen linken Handsensor 41 L bzw. rechten Handsensor 41 R, die von der linken Hand 70L respektive rechten Hand 70R eines Bedieners 70 ausgelöst wird. Wenn beide Handsensoren 41 L, 41 R ausgelöst sind, ist sichergestellt, dass der Bediener 70 das Handgerät 1 korrekt hält und der aus der Austrittsdüse 4 austretende Plasmastrom 3 im Wesentlichen weg von seinem Körper gerichtet ist. Die Erfindung sieht aus Gründen der Betriebssicherheit vor, das die Einrichtung 30 die Spannungsquelle 20 nur freigibt, so lange beide Handsensoren 41 L, 41 R ausgelöst bleiben.

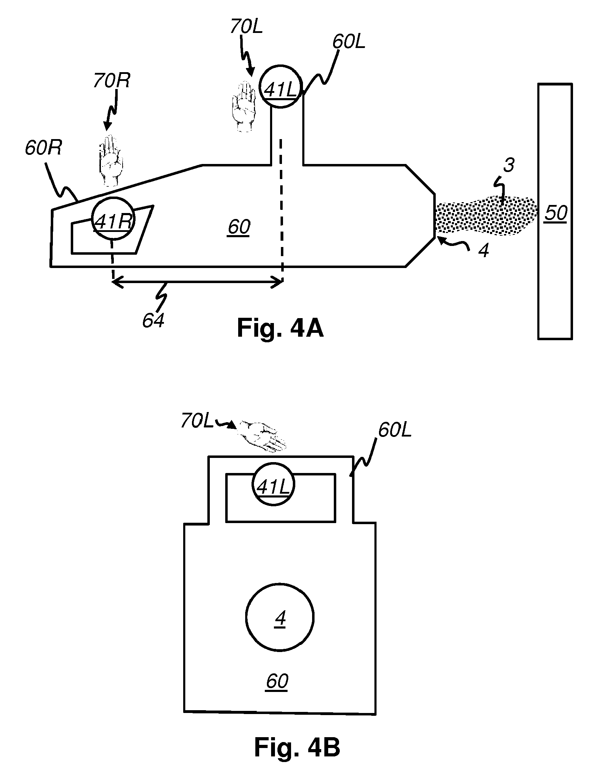

**Figur4A** zeigt eine schematische Ansicht einer weiteren Ausführungsform des Gehäuses 60 des erfindungsgemäßen Handgeräts 1. **Figur 4B** zeigt eine andere schematische Ansicht auf die Ausführungsform des Gehäuses 60 des erfindungsgemäßen Handgeräts 1 gemäß Figur 4A. Die Grundform des Gehäuses 60 entspricht dem einer Kettensäge. Die Handgriffe 60L, 60R sind jeweils als Bügel ausgebildet. Der Handgriffabstand 64 ist aus Sicherheitsgründen so bemaßt, dass eine einzige typische menschliche Hand nicht beide Handgriffe 60L, 60R gleichzeitig auslösen kann.

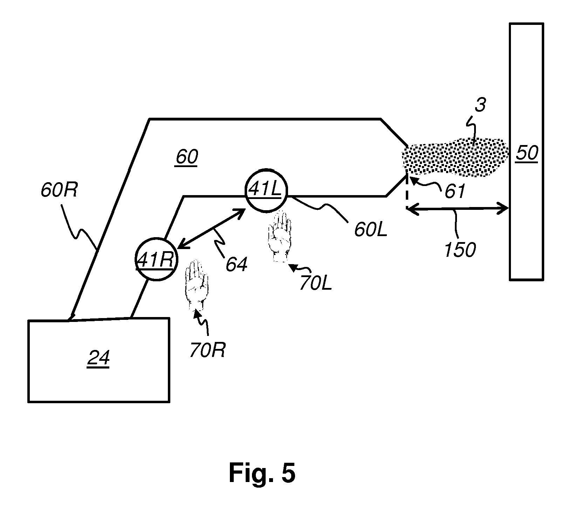

**Figur 5** zeigt eine schematische Ansicht einer weiteren Ausführungsform des Gehäuses 60 des erfindungsgemäßen Handgeräts 1. Die Grundform des Gehäuses 60 entspricht dem einer Bohrmaschine. Der rechte Handsensor 41 R fungiert gleichzeitig als Bedienelement zur Eingabe von Bediensignalen durch den Bediener 70. Er kann beispielsweise als analoger oder digitaler Druck- oder Drehknopf ausgestaltet sein. Je stärker ein Bediener 70 ihn betätigt, desto höher regelt die Einrichtung 30 beispielsweise die Plasmaleistung pro Fläche auf dem Werkstück 50. Die Einrichtung 30 bewirkt die Erhöhung der Plasmaleistung durch kombinierte Ansteuerung von Spannungsquelle 20 und Gasversorgungseinheit 10. In diesem Fall kann der linke Handsensor 41 L z.B. ein Druckknopf oder kapazitiver Sensor sein, der lediglich die Position 101 der linken Hand 70L eines Bedieners 70 erfasst. Zusätzlich kann der Handsensor 41 L auch als Bedieneranzeige 71 fungieren. Beispielsweise kann er vermittels einer integrierten Lichtquelle (nicht dargestellt) durchgängig leuchten, wenn das Handgerät 1 betriebsbereit ist, und blinken, wenn die Betriebsbereitschaft noch nicht oder nicht mehr gegeben ist oder einer der Betriebsparameter 100 einen unteren Schwellwert 102L oder oberen Schwellwert 102U länger als für die Betriebs- oder Prozesssicherheit tolerabel über- bzw. unterschreitet. Wie lange eine solche Abweichung tolerabel ist, kann der Bediener 70 oder der Hersteller des Handgeräts 1 je nach Anwendung festlegen. Die maximale tolerable Dauer einer Abweichung kann der Einrichtung 30 erfindungsgemäß in Form von Zeitintervallen Δt101 bzw.Δt102 einprogrammiert werden. Denkbar ist auch, dass sie durch einen Glättungsalgorithmus (z.B. einer Fouriertransformation) in der Einrichtung 30 implementiert ist. Um einem technisch kundigen Bediener 70 mehr direkte Einwirkungsmöglichkeiten auf die Plasmaintensität und Plasmabeschaffenheit zu belassen, ist es auch denkbar, den rechten Handsensor 41 R als Bedienelement zur Einstellung der Spannung und den linken Handsensor 41L als Bedienelement zur Einstellung des Gasstroms 13 auszugestalten.

Vorzugsweise ist im oder am Gehäuse 60 eine Batterie 24 vorgesehen, die die Spannungsquelle 20 speist. In dieser Ausführungsform ist das Handgerät nicht an Kabel und/oder Schläuche. Es kann auch ein Netzteil (nicht dargestellt) mit einem gängigen Strom- oder Starkstromstecker (nicht dargestellt) im Gehäuse 60 vorgesehen sein.

**Figur 6A** eine schematische Ansicht das Handgerät 1, wobei die Zielvorrichtung 90 dargestellt ist. Sie umfasst z.B. drei oder mehr Projektoren 92, die z.B. homogen winkelverteilt um die Austrittsdüse 4 für den Plasmastrahl 3 angeordnet sind. Jeder Projektor 92 wirft wenigstens je ein Lichtsignal 91 auf das Werkstück 50, wie in **Figur 6B** dargestellt. Im dargestellten Beispiel sind die Lichtsignale punktförmig. Denkbar sind auch andere Formen, wie beispielsweise Linien, Pfeile oder andere Symbole. Die in Figur 6B dargestellten Lichtsignale markieren den Bereich 51 auf dem Werkstück 50, auf den der Plasmastrahl 3 effektiv einwirkt, wo also eine Plasmabehandlung unter den gewünschten Betriebsbedingungen stattfindet oder stattfinden wird, falls das Plasma noch nicht gezündet worden ist. Im einfachsten Fall könnte hingegen ein einziger z.B. als Laserpointer ausgestalteter Projektor 92 einen Ort 52 markieren, der im Zentrum 53 des plasmaüberstrichenen Bereiches 51 liegt. Das Zentrum 53 des plasmaüberstrichenen Bereiches 51 liegt lotrecht unter der Mitte der Austrittsdüse 4 bzw. der Primärseite 22 der Elektrodeneinheit 2. Die Markierung des Zentrums 53 erfordert jedoch, dass der Projektor 92 unter einem anderen Winkel als die Austrittsdüse 4 auf das Werkstück 50 gerichtet. Dadurch verschiebt sich der markierte Ort 52 je nach Arbeitsabstand 105 und kann somit je nach Arbeitsabstand 105 verfälscht werden. Ein Vorteil der in Figur 6A dargestellten Ausführung der Zielvorrichtung 90 ist, dass die Projektoren 92 unter demselben Winkel wie die Austrittsdüse 4 auf das Werkstück 50 gerichtet sein können, so dass der markierte Bereich 52 nicht vom Arbeitsabstand 105 abhängt. Zudem ist zu beachten, dass typische Arbeitsabstände 105 zwischen Austrittsdüse 4 und Werkstück 50 nur 8-12 mm (siehe Figur 1) betragen. Bei derart kurzen Arbeitsabständen kann das Zentrum des plasmaüberstrichenen Bereichs 51 für einen Bediener 70 durch das Gehäuse 60 des Handgeräts 1 verdeckt werden. Eine wie in Figur 6B dargestellte exzentrische Markierung des Bereichs 51 ist hingegen besser sichtbar. Ferner kann neben der Austrittsdüse 4 eine Kamera 46 im Gehäuse 60 angeordnet sein, die das Werkstück aufnimmt und die Bilddaten an die Einrichtung 30 weiterleitet. Die Einrichtungen 30 kann Bilderkennungsverfahren auf diese Bilddaten anwenden, um Werkstücke 50 oder bestimmte Strukturen auf Werkstücken 50 zu identifizieren. Auf gleiche Weise kann die Einrichtung 30 auch die abstands- und winkelabhängige Form und relative Lage der Lichtsignale 91 auswerten, um die Lage, den Arbeitsabstand 105 und/oder die Winkelausrichtung des Handgeräts 1 bezüglich des Werkstücks 50 zu bestimmen.

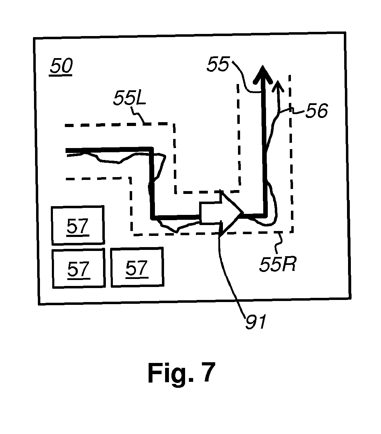

**Figur 7** zeigt eine schematische Darstellung einer erfindungsgemäß markierten und plasmazubehandelnden Soll-Trajektorie 55 auf einem Werkstück 50. Eine Soll-Trajektorie 55 kann in der Einrichtung 30 durch zeitabhängige Soll-Intervalle kinematischer Betriebsparameter 100 über dem Werkstück 50 definiert werden. Zu diesen kinematischen Betriebsparametern 100 kann insbesondere der Arbeitsabstand 105 zählen. Der Bediener 70 (siehe Figur 1 und 2) führt das Handgerät 1 auf einer Ist-Trajektorie 56 entlang der Soll-Trajektorie 55 über das Werkstück 50. Den Verlauf der Soll-Trajektorie 55 auf dem Werkstück 50 kann ein Bediener 70 beispielsweise anhand von Referenzmarken 57 auf dem Werkstück 50 ersehen bzw. die Einrichtung 30 aus den von der Kamera 46 aufgenommenen Bilddaten auswerten. Ferner wertet die Einrichtung 30 die Ist-Trajektorie 56 und ihre Abweichung von der Soll-Trajektorie 55 aus. Vermittels der Zielvorrichtung 90 kann die Soll-Trajektorie 55 zumindest abschnittsweise auf das Werkstück (50) projiziert werden. In Figur 7 ist beispielhaft Lichtsignal 91 in Form eines Pfeils in lokaler Vorschubrichtung auf das Werkstück 50 projiziert. Durch Modulation der Form oder durch Blinken dieses Pfeils können neben der Vorschubrichtung auch andere Bedienerhinweise 80 zu anderen kinematischen Betriebsparametern 100 wie der Vorschubgeschwindigkeit angezeigt werden. Denkbar ist auch, dass die von der Kamera 46 erfassten Bilddaten kontinuierlich auf einer als Bildschirm ausgestalteten Bedieneranzeige 71 angezeigt werden. Denkbar ist auch, dass die Einrichtung 30 auf einer solchen Bedieneranzeige 71 die Soll-Trajektorie 55, die Ist-Trajektorie 56, das Zentrum 53 des aktuell plasmaüberströmten Bereichs 52 und/oder sonstige Bedienerhinweise 80 mit den Bilddaten überlagert darstellt. Ebenso kann der Verlauf einer linken und rechten Toleranztrajektorie 55L bzw. 55R erfasst und dargestellt werden. Dies hat den Vorteil, dass ein Bediener 70 alle für die Plasmabehandlung notwendige Information in übersichtlicher und verknüpfter Form auf einer solchen Bedieneranzeige 71 ersehen kann. Auf dem Werkstück 50 projizierte Lichtsignale können dagegen vom Gehäuse 60 verdeckt und von der Leuchtkraft des Plasmastroms 3 überlagert werden.

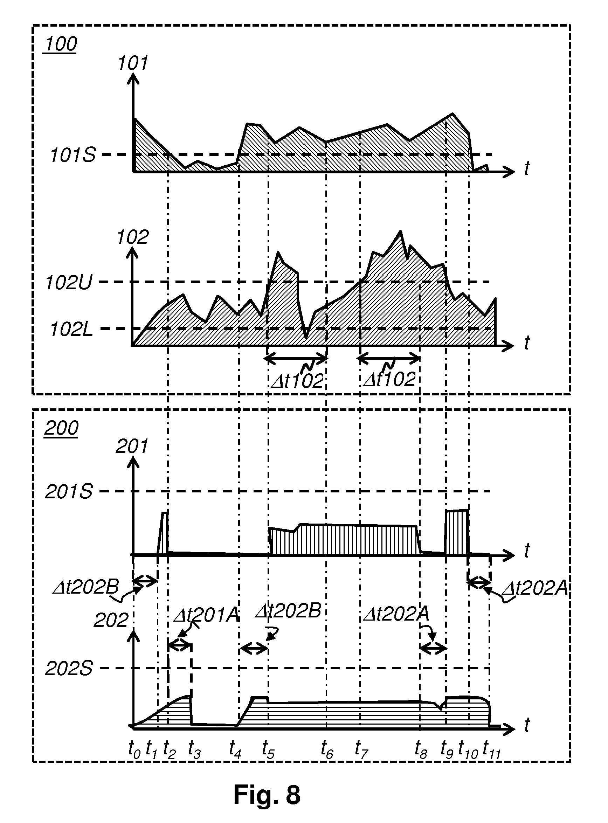

In **Figur 8** ist beispielhaft ein erfindungsgemäßer Verfahrensgang zur Plasmabehandlung von Werkstücken 50 anhand schematischer Zeitdiagramme für verschiedene Betriebsparameter 100 und Steuersignale 200 dargestellt. Die exemplarisch dargestellten Betriebsparameter 100 sind eine Position 101 eines Bedieners 70 relativ zur Plasmaquelle 5 sowie ein Druck 102 in der Plasmaquelle 5. Die exemplarisch dargestellten Steuerparameter 200 sind das Steuersignal 201 für die Spannungsquelle 20 sowie das Steuersignal 202 für die Gasversorgungseinheit 10. Die Erfassung von Betriebsparametern 100 und die Ausgabe von Steuersignalen 200 erfolgt synchronisiert, so dass sich die Zeitpunkte t₀, ..., t₁₁ auf alle Zeitdiagramme beziehen.

Im Zeitpunkt t₀ betätigt ein Bediener 70 eine Bedienersensoreinrichtung 41 eines erfindungsgemäßen Handgeräts 1. Die Bedienersensoreinrichtung 41 übermittelt der Einrichtung 30 den Betriebsparameter 100 'Position 101' und stellt fest, dass seine Signalstärke über einem vorgegebenen unteren Positions-Schwellwert 101 S liegt. Daraufhin gibt sie ein Steuersignal 202 an die Gasversorgungseinheit 10, welches den Hochlauf des Gasstroms 13 bewirkt.

Im Zeitpunkt t₁, nach Ablauf einer Vorlaufzeitspanne Δt202B für die Stabilisierung des Gasstroms 13, ermittelt die Einrichtung 30, dass der Druck 102 innerhalb des von einem unteren Druck-Schwellwert 102L und oberen Druck-Schwellwert 102U definierten Soll-Intervalls liegt. Daraufhin gibt sie ein Steuersignal 201 an die Spannungsquelle 20 aus, das z.B. proportional zur an der Elektrodeneinheit 2 angelegten Spannung ist.

Im Zeitpunkt t₂lockert der Bediener 70 sein Griff um die eine Bedienersensoreinrichtung 41, so dass der Betriebsparameter 100 'Position 101' unter den unteren Positions-Schwellwert 101 S fällt. Sofort stoppt die Einrichtung 30 aus Sicherheitsgründen das Steuersignal 201 an die Spannungsquelle 20 und damit die Spannung, so dass der Plasmastrom 3 erlischt.

Im Zeitpunkt t₃, nach Ablauf eines Nachlaufzeitspanne Δt202A, stoppt die Einrichtung 30 ebenfalls das Steuersignal 202 für den Gasstrom 13. Das Nachlaufen des Gasstroms 13 kühlt die Plasmaquelle 5.

Im Zeitpunkt t₄ greift der Bediener 70 das Handgerät 1 wieder richtig, so dass der Betriebsparameter 100 'Position 101' den unteren Positions-Schwellwert 101 S wieder überschreitet. Daraufhin gibt die Einrichtung 30 zunächst den Gasstrom 13 und nach Ablauf eines neuerlichen eines Vorlaufzeitspanne Δt202B, im Zeitpunkt t₅, die Spannung wieder frei, so dass erneut ein Plasmastrahl 3 erzeugt wird.

Zwischen Zeitpunkt t₅ und im Zeitpunkt t₆ kommt es zu kurzzeitigen Schwankungen im Druck 102, die jedoch als tolerabel definiert sind, weil sie das Soll-Intervall nicht länger als eine voreingestelltes Zeitintervall Δt102 verlassen. Die Einrichtung 30 lässt die Steuersignale 201, 202 für Spannungsquelle 20 und Gasversorgungseinheit 10 unverändert.

Zwischen Zeitpunkt t₇ und im Zeitpunkt t₉ übersteigt der Druck 102 den oberen Druck-Schwellwert 102U länger als das Intervall Δt102. Daraufhin stoppt die Einrichtung 30 im Zeitpunkt t₈ das Steuersignal 201 für die Spannungsquelle 20. Auch das Steuersignal 202 für die Gasversorgungseinheit 10 würde von der Einrichtung 30 nach Ablauf einer Nachlaufzeit Δt202A gestoppt, wenn im Zeitpunkt t₉ der Druck 102 nicht wieder im Soll-Intervall läge. Im vorliegenden Beispiel läuft der Gasstrom 13 also weiter. Im Zeitpunkt t₉ wird die Spannungsquelle 20 wieder angesteuert, um einen Plasmastrom 3 zu zünden.

Im Zeitpunkt t₁₀ beendet der Bediener 70 die Plasmabehandlung und legt das Handgerät 1 ab. Die Einrichtung 30 unterbricht das Steuersignal 201 für die Spannungsquelle 20 sofort und im Zeitpunkt t₁₁, nach einer neuerlichen Nachlaufzeit Δt202A, auch das Steuersignal 202 für die Gasversorgungseinheit 10.

Das Verfahren kann analog mit jeder Kombination von Betriebsparametern 100 durchgeführt werden. Ebenso können Steuerschwellwerte 201 S und 202S für die Steuersignale 201 bzw. 202 definiert werden, bei deren Überschreiten die Einrichtung 30 die Spannungsquelle 20 und ggf. die Gasversorgungseinheit 10 abschaltet.

### Bezugszeichenliste:

- 1: Handgerät
- 2: Elektrodeneinheit
- 3: Plasmastrom
- 4: Austrittsdüse
- 5: Plasmaquelle
- 10: Gasversorgungseinheit
- 11: Gasversorgungskanal
- 13: Gasstrom
- 20: Spannungsquelle
- 21: elektrische Leitung
- 22: Primärseite
- 23: Sekundärseite
- 24: Batterie
- 30: Einrichtung
- 31, 32, 33, 34, 35, 36, 37: Datenleitungen
- 38: interner Taktgeber
- 40: Sensorsystem
- 41: Bedienersensoreinrichtung
- 41L: linker Handsensor
- 41R: rechter Handsensor
- 42: Drucksensor
- 43: Durchflusssensor
- 44: Temperatursensor
- 45: kinematischer Sensor
- 46: Kamera
- 50: Werkstück
- 51: Bereich
- 52: Ort
- 55: Soll-Trajektorie
- 55L: linke Toleranztrajektorie
- 55R: rechte Toleranztrajektorie
- 56: Ist-Trajektorie
- 55: Soll-Trajektorie
- 56: Ist-Trajektorie
- 57: Referenzmarke
- 60: Gehäuse
- 60L: linker Handgriff
- 60R: rechter Handgriff
- 63: Mitte
- 64: Handgriffabstand
- 70: Bediener
- 71: Bedieneranzeige
- 70L: linke Hand
- 70R: rechte Hand
- 80: Bedienerhinweise
- 90: Zielvorrichtung
- 91: Lichtsignale
- 100: Betriebsparameter
- 101S: maximaler Schwellwert
- 102L: unterer Schwellwert
- 102U: oberer Schwellwert
- 105: Arbeitsabstand
- Δt101, Δt102: Zeitintervall
- Δt201A: Vorlaufzeitspanne
- Δt201: BNachlaufzeitspanne
- 200, 201, 202: Steuersignale
- 201S, 202S: Steuerschwellwert
- 300: Datenbank
- t: Zeit

## Patentansprüche

1. Handgerät (1) zur Plasmabehandlung von Werkstücken (50) mit einem Gehäuse (60) zur Aufnahme einer Plasmaquelle (5), einer Gasversorgungseinheit (10) zur Zuführung eines Gasstroms (13) zur Plasmaquelle (5) und einer Spannungsquelle (20), die über mindestens eine elektrische Leitung (21) mit einer Elektrodeneinheit (2) der Plasmaquelle (5) zur Erzeugung eines Plasmastroms (3) verbunden ist und einer Austrittsdüse (4) des Gehäuses (60), aus der der Plasmastrom (3) auf ein Werkstück (50) richtbar ist,
**gekennzeichnet durch**
- ein Sensorsystem (40) zur Erhebung von Betriebsparametern (100), wobei das Sensorsystem (40) wenigstens eine Bedienersensoreinrichtung (41) aus einem linken Handsensor (41 L) und einen rechten Handsensor (41 R) zur Erhebung einer Position einer rechten Hand (70R) bzw. einer linken Hand (70L) eines Bedieners (70) am Gehäuse (60), und wobei damit eine Position (101) des Bedieners (70) relativ zur Plasmaquelle (5) erfassbar ist und wenigstens einen Drucksensor (42) zur Erhebung eines Drucks (102) in der Gasversorgungseinheit (10) umfasst; und
- eine Einrichtung (30), die mit dem Sensorsystem (40) zur synchronisierten Erfassung der erhobenen Betriebsparameter (100) und zumindest mit der Spannungsquelle (20) zu ihrer Regelung über Datenleitungen (31, 32, 36) kommunikativ verbunden ist.

2. Handgerät (1) nach Anspruch 1, wobei das Sensorsystem (40) zusätzlich einen der folgenden Sensoren umfasst:
- mindestens einen Durchflusssensor (43) zur Erhebung einer Flussrate des Gasstroms (13) durch die Plasmaquelle (5), der in der Plasmaquelle (5) oder in der Gasversorgungseinheit (10) angeordnet ist;
- mindestens einen Temperatursensor (44) zur Erhebung einer Temperatur der Plasmaquelle (5), der in oder an der Plasmaquelle (5) angeordnet ist;
- mindestens eine Kamera (46) zur Erfassung zumindest eines Bereichs (51) des Werkstücks (50) und/oder
- mindestens einen kinematischer Sensor (45) zur Erhebung zumindest einer kinematischen Größe des Handgeräts (1) relativ zum Werkstück (50).

3. Handgerät (1) nach Anspruch 1 oder 2, wobei die Einrichtung (30) oder das Sensorsystem (40) zur Durchführung je eines Soll-Ist-Abgleichs für jeden vom Sensorsystem (40) erhobenen Betriebsparameter (100) ausgebildet ist.

4. Handgerät (1) einem der vorangegangenen Ansprüche, wobei das Handgerät (1) eine Zielvorrichtung (90) umfasst, die zur Markierung eines Bereichs (51) auf dem Werkstück (50) dient, auf den der Plasmastrom (3) gerichtet ist.

5. Handgerät (1) nach Anspruch 3, wobei mittels mindestens einer Bedieneranzeige (71) des Handgeräts (1) optische, akustische und/oder vibratorische Bedienerhinweise (80) bezüglich des Betriebszustandes des Handgeräts (1) ausgebbar sind.

6. Handgerät (1) nach einem der vorangegangenen Ansprüche, wobei die Einrichtung (30) mit einer Datenbank (300) zur Speicherung von Betriebsparametern (100) und/oder aus Betriebsparametern (100) abgeleiteten und/oder auf den Bediener (70) bezogenen Metadaten kommunikativ verbunden ist.

7. Handgerät (1) nach einem der vorangegangenen Ansprüche, wobei die Einrichtung (30) über eine Datenleitung (37) mit der Gasversorgungseinheit (10) zur Regelung des Gasstroms (13) kommunikativ verbunden ist, wobei die Gasversorgungseinheit (10) ein regelbares Gebläse und/oder eine Gasdruckflasche mit einem regelbaren Ventil ist.

8. Verfahren zur Plasmabehandlung eines Werkstücks (50), **gekennzeichnet durch** die folgenden Schritte:
- dass mit einer Plasmaquelle (5) eines Handgeräts(1) zur Plasmabehandlung ein Plasmastrom (3) erzeugt wird und der erzeugte Plasmastrom (3) auf ein Werkstück (50) gerichtet wird;
- dass mittels eines Sensorsystems (40) mindestens zwei Betriebsparameter (100) erhoben werden, wobei zumindest mittels wenigstens einer Bedienersensoreinrichtung (41) des Sensorsystems (40), die aus einem linken Handsensor (41 L) und einen rechten Handsensor (41 R) besteht, eine Position (101) eines Bedieners (70) relativ zur Plasmaquelle (5) erfasst wird und mittels wenigstens eines Drucksensors (42) des Sensorsystems (40) ein Druck (102) in einer Gasversorgungseinheit (10) der Plasmaquelle (5) erfasst wird;
- dass die erhobenen Betriebsparameter (100) an eine Einrichtung (30) synchronisiert übermittelt werden; und
- dass **durch** die Einrichtung (30) anhand der erhobenen Betriebsparameter (100) Steuersignale (200) zum Freigeben und/oder Regeln zumindest einer Spannungsquelle (20) der Plasmaquelle (5) erzeugt werden.

9. Verfahren nach Anspruch 8, wobei mittels des Sensorsystems (40) zusätzlich mindestens einer der folgenden Betriebsparameter (100) erhoben wird:
- mindestens eine Flussrate des Gasstroms (13) mittels mindestens eines in der Plasmaquelle (5) oder in der Gasversorgungseinheit (10) angeordneten Durchflusssensors (43);
- mindestens eine Temperatur der Plasmaquelle (5) mittels mindestens eines in oder an der Plasmaquelle (5) angeordneten Temperatursensors (44);
- ein optisches Abbild zumindest eines Bereiches (51) des Werkstücks (50) mittels einer Kamera (46) des Handgeräts (1);
- mindestens einen kinematischen Betriebsparameter (100) des Handgeräts (1) relativ zum Werkstück (50) mittels mindestens eines am Handgerät (1) angeordneten kinematischen Sensors (45).

10. Verfahren nach Anspruch 8 und 9, wobei für die erhobenen Betriebsparameter (100) je ein Soll-Ist-Abgleich durchgeführt wird, und wobei die Einrichtung (30) Steuersignale (200) erzeugt, wenn beim jeweiligen Soll-Ist-Abgleich der entsprechende Betriebsparameter (100) länger als ein jeweiliges Zeitintervall (Δt101, Δt102) entweder einen jeweiligen minimalen oder maximalen Schwellwert (101S) unterschreitet respektive überschreitet oder innerhalb oder außerhalb eines jeweiligen Soll-Intervalls liegt, das durch einen jeweiligen oberen Schwellwert (102U) und einen jeweiligen unteren Schwellwert (102L) definiert wird.

11. Verfahren nach Anspruch 8, wobei dem Bediener (70) vermittels einer Bedieneranzeige (71) des Handgeräts (1) Bedienerhinweise (80) bezüglich der Betriebsbereitschaft des Handgeräts (1), zumindest eines erhobenen Betriebsparameters (100) und/oder des Überschreitens und Unterschreitens von oberen Schwellwerten (102U) respektive unteren Schwellwerten (102L) mindestens eines der erhobenen Betriebsparameter (100) angezeigt wird und wobei mittels einer Zielvorrichtung (90) des Handgeräts (1) mit wenigstens einem Projektor (92) mindestens ein Lichtsignal (91) auf das Werkstück (50) projiziert wird, um einen Ort (52) und/oder einen Bereich (51) auf dem Werkstück (50) zu markieren, auf den der Plasmastrom (3) gerichtet ist.

12. Verfahren nach Anspruch 11, wobei durch zeitabhängige Soll-Intervalle kinematischer Betriebsparameter (100) eine Soll-Trajektorie (55) über dem Werkstück (50) definiert wird, wobei der Bediener (70) das Handgerät (1) auf einer Ist-Trajektorie (56) entlang der Soll-Trajektorie (55) über das Werkstück (50) führt und wobei die Zielvorrichtung (90) die Soll-Trajektorie (55) zumindest abschnittsweise auf das Werkstück (50) projiziert.

13. Verfahren nach Anspruch 8, wobei die Einrichtung (30) Steuersignale (201) an die Spannungsquelle (20) nur ausgibt, solange kein erhobener Betriebsparameter (100) länger als ein jeweiliges Zeitintervall (Δt101, Δt102) außerhalb eines jeweiligen Soll-Intervalls liegt oder einen jeweiligen minimalen oder maximalen Schwellwert (101 S) unterschreitet oder respektive überschreitet.

14. Verfahren nach Anspruch 8, wobei die Einrichtung (30) Steuersignale (202) für eine Gasversorgungseinheit (10) der Plasmaquelle (5) erzeugt und die Spannungsquelle (20) und die Gasversorgungseinheit (10) in Abhängigkeit der jeweils in der Gasversorgungseinheit (10) und der Spannungsquelle (20) vorherrschenden Betriebsparameter regelt.

15. Verfahren nach Anspruch 8, wobei die Einrichtung (30) vermittels eines internen Taktgebers (38) die Spannungsquelle (20) und die Gasversorgungseinheit (10) derart zeitlich abgestimmt steuert, dass der Gasstrom (13) die Plasmaquelle (5) während einer Vorlaufzeitspanne (Δt202B) vor einer Freigabe der Spannungsquelle (20) und/oder während einer Nachlaufzeitspanne (Δt202A) nach Abschalten der Spannungsquelle (20) durchfließt.
